# EUROPEAN PATENT APPLICATION

(11) **EP 4 474 472 A1**
(43) Date of publication of application: **11.12.2024**
(21) Application number: 23382541.3
(22) Date of filing: 05.06.2023
(51) Int. Cl.: C12N 1/20, C05F 11/08, C12N 11/00

(54) **COMPOSITION AND METHOD FOR CARBON SEQUESTRATION IN SOIL, WHICH COMPRISES THE BACTERIA MICROBACTERIUM MARITYPICUM**

(71) Applicant: Haza Roraima Eco, 04120 La Cañada (Almeria) (ES)
(72) Inventor: DEL ÁGUILA CAPEL, Francisco Javier, 04120 La Cañada (Almeria) (ES); MEZA TAPIA, Carlos Andrés, 04120 La Cañada (Almeria) (ES)
(74) Representative: Hoffmann Eitle

(57) **Abstract**

The present invention describes a composition suitable for increasing the fixation of carbon in soil comprising the bacterium *Microbacterium maritypicum.* The present invention also describes uses of said composition and methods which comprise applying the composition of the invention on soil.

## Description

### Technical Field of the Invention

The present invention belongs to the field of agriculture and environmental conservation. In particular, the present invention relates to isolated and biologically pure microorganisms applicable in agriculture and environmental conservation, more particularly, to a method for fixing carbon in soil by means of a compound comprising the bacterium *Microbacterium maritypicum,* a method for increasing water retention in soil by means of said compound, and a composition comprising *Microbacterium maritypicum.*

### Background of the Art

Carbon is a plant macronutrient which can be absorbed mainly as CO₂ through the aerial part of plants. Root absorption allows incorporating carbon in the plant as bicarbonate HCO₃, the mobility of which is high. It is also incorporated in the organic fraction of the plant in the form of carboxyl functional group (-COOH), by means of carboxylation reactions.

Other ways of transport are:
- Intercellular, as CO₂.
- Through the phloem and xylem, as organic acids.
- Through the phloem, as disaccharide.

Furthermore, it can be stored in storage organs as carbohydrates, and it is also incorporated in the organic fraction of the plant through proteins or fats.

Carbon compounds from plants and algae that existed long ago form fossil fuels, such as coal and natural gas, that are used today as energy sources. When these fossil fuels are burned, carbon dioxide is released into the atmosphere, resulting in increasing levels of atmospheric CO₂. This increase in CO₂ levels affects the earth's climate and is a major environmental concern worldwide.

Carbon is part of the main structural building blocks of plant macromolecules, furthermore it is also part of very important functional groups that are involved in enzymatic processes. Its assimilation by means of oxidation and reduction processes should be highlighted as the most relevant biochemical properties. Agronomically speaking, carbon is of vital importance in the correct formation of the soil structure and in promoting good root development of the crop. On the other hand, in relation to other macronutrients, this element promotes the appearance of a microbiome that is beneficial for plant health, establishing symbiotic relationships between them, referred to as the rhizosphere.

Climate change poses a major threat to food security due to its significant negative impact on agriculture, livestock, and fisheries, entailing reductions in yields, biological migration, and loss of ecosystem services which ultimately means a reduction in agricultural income and an increase in food prices. The air CO₂ level is close to 0.04% (400 ppm), having increased by 148% since pre-industrial times. Its concentrations vary between 300 ppm and 550 ppm, depending on whether measurement is performed in rural or urban environments (FAO, 2007).

It should be noted that the notation system used to define the air CO₂ level as close to 0.04% is the Anglo-Saxon system, which uses a decimal point to delimit the beginning of decimal places. This notation will be followed throughout the text of the document.

The fixation or absorption of CO₂ in soil can help mitigate these problems while offering a solution to one of the main causes of global warming. Therefore, there is a need to implement a series of CO₂ fixation practices, suggested to achieve the maximum potential for climate change mitigation and adaptation and food productivity (FAO, 2007). The present invention addresses the problem of incorporating means which allow improving the fixation of CO₂ in soil.

### Summary of the Invention

A first aspect of the invention relates to a composition, preferably an aqueous or lyophilized composition, suitable for increasing the fixation of carbon in soil comprising the bacterium *Microbacterium maritypicum.*

In a preferred embodiment of the invention, the bacterium *Microbacterium maritypicum* is present in the composition of the invention in an amount of between 1×10² and 5.6×10⁸ cfu/g (colony-forming units per gram).

In another preferred embodiment of the first aspect of the invention, the composition comprises the bacterium *Microbacterium maritypicum,* preferably in an amount of between 1×10² and 5.6×10⁸ cfu/g, wherein said bacterium is dissolved or dispersed in a medium characterized in that it comprises one or more peptones, extract of one or more yeasts, sodium chloride, magnesium chloride, calcium chloride, and one or more saccharides, preferably monosaccharides or disaccharides, more preferably brown sugar, wherein said components are comprised in an aqueous medium such as water, preferably sterilized water.

In a preferred embodiment, the composition comprises the bacterium *Microbacterium maritypicum,* preferably in an amount of between 1×10² and 5.6×10⁸ cfu/g, wherein said bacterium is dissolved or dispersed in a medium characterized in that the medium comprises, in percentage by weight with respect to the total weight of the medium (%w/w), between 0.06 and 6% of one or more peptones, between 0.04 and 4% of extract of one or more yeasts, between 0.15 and 15% of sodium chloride, between 0.07 and 7% of magnesium chloride, between 0.05 and 5% of one or more saccharides, preferably brown sugar, between 0.01 and 1% of calcium chloride, and an aqueous medium such as water, preferably sterilized water, until completing 100%.

In another preferred embodiment, the composition comprises the bacterium *Microbacterium maritypicum,* preferably in an amount of between 1×10² and 5.6×10⁸ cfu/g, wherein said bacterium is dissolved or dispersed in a medium, characterized in that the medium comprises ±20% of 0.578% of one or more peptones, i.e., between a 0.4624 and 0.6936% of one or more peptones, ±20% of 0.385% of extract of one or more yeasts, ±20% of 1.445% of sodium chloride, ±20% of 0.674% of magnesium chloride, ±20% of 0.096% of calcium chloride, ±20% of 0.482% of one or more saccharides, preferably monosaccharides or disaccharides, more preferably brown sugar, and an aqueous medium such as water, preferably sterilized water, until completing 100% (%w/w).

In another preferred embodiment of the invention, the medium in which the bacterium *Microbacterium maritypicum* is dissolved or dispersed, preferably in an amount of between 1×10² and 5.6×10⁸ cfu/g, further comprises ferric citrate, sodium sulfate, potassium chloride, sodium bicarbonate, potassium bromide, strontium chloride, boric acid, sodium fluoride, ammonium nitrate, and disodium phosphate, wherein said components are comprised in an aqueous medium such as water, preferably sterilized water.

In another more preferred embodiment of the invention, the medium in which the bacterium *Microbacterium maritypicum* is dissolved or dispersed, preferably in an amount of between 1×10² and 5.6×10⁸ cfu/g, comprises, in percentage by weight with respect to the total weight of the medium (%w/w), between 0.1 and 1.2% of one or more peptones, between 0.01 and 1 % of extract of one or more yeasts, between 0.001 and 0.1% of ferric citrate, between 0.2 and 8% of sodium chloride, between 0.1 and 5% of magnesium chloride, between 0.05 and 3% of sodium sulfate, between 0.01 and 0.15% of calcium chloride, between 0.005 and 0.5% of potassium chloride, between 0.002 and 0.2% of sodium bicarbonate, between 0.001 and 0.5% of potassium bromide, between 0.0002 and 0.03% of strontium chloride, between 0.0002 and 0.03% of boric acid, between 0.0001 and 0.003% of sodium fluoride, between 0.00005 and 0.002% of ammonium nitrate, between 0.0001 and 0.01% of disodium phosphate, optionally between 0.05 and 5% of one or more saccharides, preferably monosaccharides or disaccharides, more preferably brown sugar, and an aqueous medium such as water, preferably sterilized water, until completing 100%.

In an even more preferred embodiment, the medium in which the bacterium *Microbacterium maritypicum* is dissolved or dispersed, preferably in an amount of between 1×10² and 5.6×10⁸ cfu/g, consists of ±20% of 0.5758% of one or more peptones, ±20% of 0.1248% of extract of one or more yeasts, ±20% of 0.0144% of ferric citrate, ±20% of 1.9289% of sodium chloride, ±20% of 0.8637% of magnesium chloride, ±20% of 0.3023% of sodium sulfate, ±20% of 0.1439% of calcium chloride, ±20% of 0.04798% of potassium chloride, ±20% of 0.01919% of sodium bicarbonate, ±20% of 0.0067% of potassium bromide, ±20% of 0.0029% of strontium chloride, ±20% of 0.0024% of boric acid, ±20% of 0.00029% of sodium fluoride, ±20% of 0.00017% of ammonium nitrate, ±20% of 0.00096% of disodium phosphate, optionally ±20% of 0.5% of one or more saccharides, preferably monosaccharides or disaccharides, more preferably brown sugar, and an aqueous medium such as water, preferably sterilized water, until completing 100% (%w/w).

In another preferred embodiment, water has been extracted from the medium of the composition of the invention in which the bacterium *Microbacterium maritypicum* is dissolved or dispersed, preferably by means of lyophilization or drying, preferably in an amount of between 1×10² and 5.6×10⁸ cfu/g.

A second aspect of the invention relates to the use of the composition of the invention, as defined in the first aspect of the invention or in any of the preferred embodiments thereof, for fixing carbon in soil.

A preferred embodiment of the second aspect of the invention, as defined in the first aspect of the invention or in any of the preferred embodiments thereof, relates to the use of the composition of the invention for increasing water retention in soil.

A third aspect of the invention relates to a method for increasing the fixation of carbon in soil which comprises applying on said soil the composition of the invention.

A more preferred embodiment of the third aspect of the invention relates to a method wherein between 1 and 100 liters of the composition of the invention are applied on one hectare of soil.

In an even more preferred embodiment, between 1 and 100 liters of the composition of the invention are diluted in between 1000 and 100000 liters of water prior to the application thereof on one hectare of soil, preferably diluted in between 3000 and 30000 liters of water prior to the application thereof on one hectare of soil.

In an even more preferred embodiment of the invention, the application of the composition on said soil is performed by an irrigation method consisting of watering, spray irrigation, gravity irrigation, drip irrigation, flood irrigation, tractor irrigation, cistern irrigation, sprinkler irrigation or underground irrigation.

### Brief Description of the Figures

Figure 1 shows the coupling cylinders and equipment used in cell respiration assays. The infrared sensor arm which performs the measurement is observed.
Figure 2 shows scanning electron microscopy images for a control soil (Figure 2A) and a soil treated by means of the described method (Figure 2B); both images are at the same scale.
Figure 3 shows the percentages of moisture and grain size distribution for treated soil and control soil samples. Soils with different crops are observed in both cases.
Figure 4 shows the results of CO₂ respiration (mmol m⁻² s⁻¹) for treated soil and control soil samples in conditions of being completely dried and having moisture after 24 h.
Figure 5 shows the results of CO₂ respiration (mmol m⁻² s⁻¹) for treated soil and control soil samples in conditions of being completely dried and having moisture after 24 h.
Figure 6 shows the differences of metabolites (mg metabolite/g soil) detected in Baby leaf lettuce crops for control soils (untreated) and treated soils.

### Description of the Invention

### Definitions

It should be taken into account that, as used herein, the singular forms "a", "a" and "the", include plural references unless the context clearly indicates otherwise. Furthermore, unless otherwise indicated, the term "at least" preceding a series of elements should be understood to refer to each element in the series. Those skilled in the art will recognize, or will be able to determine using no more than routine experimentation, many equivalents to the specific embodiments of the invention described herein. Said equivalents are intended to be comprised in the present invention.

It is observed that the term "about", as it is used herein, refers to +/- 30%, preferably +/- 20%, preferably +/- 15%, more preferably +/- 10%, of the indicated value.

As used herein, the term "and/or" among multiple listed elements is understood to comprise both individual and combined options. For example, when two elements are joined by "and/or", a first option refers to the applicability of the first element without the second. A second option refers to the applicability of the second element without the first. A third option refers to the applicability of the first and second elements together. It is understood that any of these options fall within the meaning, and therefore satisfy the requirement of the term "and/or" as used herein. It is also understood that the simultaneous applicability of more than one of the options falls within the meaning, and therefore satisfies the requirement of the term "and/or".

Throughout this specification and the following claims, unless the context requires otherwise, the word "comprise", and variations such as "comprises" and "comprising", will be understood to mean the inclusion of an integer or step or group of established integers or steps, but not the exclusion of any other integer or step or group of integers or steps. When used herein, the term "comprising" may be substituted with the term "containing" or "including" or sometimes, when used herein, with the term "having". Any of the aforementioned terms (comprising, containing, including, including, having), provided that it is used herein in the context of an aspect or embodiment of the present invention, can be substituted with the term "consisting of", although it is less preferred.

When used herein, "consisting of" excludes any element, step, or ingredient not specified in the claim element. When used herein, "consisting essentially of" does not exclude materials or steps that do not materially affect the basic novel features of the claim.

The term *"Microbacterium maritypicum"* refers to the bacterium *Microbacterium maritypicum* discovered by ZoBell and Upham in 1944, also known as *Flavobacterium marinotypicum* or *Microbacterium marinotypicum.* The taxonomy classification is:
Kingdom: Bacterium
Phylum: Actinomycetota (actinobacteria)
Class: Actinomycetes (high G+C Gram-positive bacterium)
Order: Micrococcales
Family: Microbacteriaceae
Genus: *Microbacterium*
Specie: *Microbacterium marinotypicum*

Therefore, the term *"Microbacterium maritypicum"* refers to all the strains of said bacterium *Microbacterium maritypicum,* such as, for example, but not limited to, strains MF109, DSM 12512, UBA6732, KMM3898, KCCM43203, S5-5, YL-1, G10, or H11, among many other strains, preferably strain DSM 12512, which can be obtained from the German Collection of Microorganisms and Cell Cultures, Leibniz Institute DSMZ.

The term "composition of the invention" refers to a composition suitable for favoring the development of microorganisms, particularly bacteria. The composition of the invention comprises the microorganism *Microbacterium maritypicum* itself. It should be noted that the composition is preferably defined as an aqueous composition which, in addition to the microorganism *Microbacterium maritypicum* itself, comprises probiotic chemical molecules and compounds such as, for example, but not limited to, salts, amino acids, nucleotides, lipids, vitamins, carbohydrates, and acids, among others, as illustrated throughout the present invention. Moreover, the composition of the invention may not contain water and may be, for example, lyophilized.

The term "atmospheric carbon" in the context of the invention refers to carbon dioxide (CO₂) found in the earth atmosphere. It is one of the gases that contributes most to the greenhouse effect and global warming. Atmospheric carbon is produced through natural processes such as respiration of living organisms and volcanic activity, but it is also released in large quantities as a result of human activities such as the burning of fossil fuels or deforestation.

The term "fixation of carbon in soil" in the context of the present invention refers to the capture or absorption of atmospheric carbon, or CO₂, by microorganisms inhabiting said soil. It should be noted that the bacterium *Microbacterium maritypicum* can contribute to this fixation of carbon in soil both directly by means of respiration and indirectly by helping the flora present in said soil to flourish, particularly by promoting those bacteria that help to capture carbon from the atmosphere and are beneficial for the plants.

The term "water retention in soil" in the context of the present invention refers to the capacity of a soil to retain water in its more superficial layers, such that plant and crop roots have access to said water or moisture before it seeps into deeper layers beyond their reach. Water retention in soil can depend on soil structuring, as well as on soil type, and said water can come from different sources such as, for example, but not limited to, irrigation water or rainwater.

The unit "cfu/g" is a measurement commonly used in microbiology to quantify the amount of bacteria or microorganisms present in a sample. "cfu" is the abbreviation for "colony-forming units", and "g" refers to grams, which is the unit of mass used in measurement. The unit cfu/g is preferably calculated by counting the number of bacterial colonies formed on an agar plate after culturing the sample. Each colony originates from a single bacterial cell or group of bacterial cells and is used as a measurement of the bacterial density in the original sample. Thus, the unit cfu/g indicates the number of viable bacteria present in a sample in a given amount of mass (grams).

Each embodiment described herein is contemplated as applicable to each of the other described embodiments. Therefore, all combinations of the various elements described herein are within the scope of the invention. It should also be understood that, unless clearly indicated otherwise, in any method claimed herein which includes more than one step or action, the order of the steps or actions of the method is not necessarily limited to the order in which the steps or actions are recited in the method.

### Description

The major problem in modern agriculture is the gradual and increasingly accelerated loss of soil structure. This leads to a deterioration of the soil microbiome, and accordingly to a loss of genetic expression in crops, making them increasingly dependent on external inputs. The major challenge in current agriculture is not only to protect, but also to improve moisture and nutrient retention.

On the other hand, atmospheric carbon, in the form of CO₂, is one of the greenhouse gases that contribute most to global warming and climate change. Therefore, the increase in atmospheric carbon levels is one of the main environmental concerns today, due to its impact on the global climate and on the health and well-being of ecosystems and life on Earth.

In this invention, see examples, a tool which improves the fixation of carbon in soil is shown. This has different benefits for crop land:
- Soil structuring,
- Greater water retention,
- Improvement of microbiota,
- Reduction of metabolites harmful for crops,
- Increase of metabolites beneficial for crops, and
- Use as fertilizer for the soil.

This ultimately translates into a higher production yield, water savings, and cost reductions.

Furthermore, the fixation of atmospheric carbon in soil contributes to the reduction of global warming and climate change. Finding compositions and methods capable of capturing said carbon present in the atmosphere and returning it to the soil therefore has an increasingly relevant ecological and environmental importance.

The examples below provide the technical results which support what has been indicated above obtained from different crops and areas in the south of Spain. To generate these results, a compound referred to as "compound of the invention", which comprises the bacterium *Microbacterium maritypicum,* has been used. This compound has been developed for the purpose of regenerating halophilic organisms native to the soil, which produce peptides that are utilized by the crop and the microbiome that encompasses the root. These organisms are similar to the primordial organisms that brought about habitable soils for other microorganisms and plant species, from rocks and extreme salinity conditions. These microorganisms are capable of transforming complex salts which cannot be utilized by the rhizosphere into nutrients that can be assimilated by it, promoting the development of beneficial microorganisms which, among other functions, fix CO₂ in soil in organic forms, thereby improving soil structuring and quality.

Regenerating native halophilic soil organisms with the compound of the invention has an effect similar to that of fertilizing the soil, given that this microbiota, at the end of its life cycle, becomes fertilizer for the soil itself, both in terms of carbon capture and in terms of metabolites and organic and biological compounds available in the soil.

Furthermore, in the examples in which the composition of the invention was applied, neither bactericides nor fungicides were applied on the soil or irrigation water, to prevent a detrimental effect on the bacterium *Microbacterium maritypicum.* The fact that neither bactericides nor fungicides are used furthermore makes this invention environmentally friendly, since soils treated with the composition of the invention can dispense with these chemicals that are harmful for the natural microbiota of the soil.

In Example 2, the amount of carbon fixed in soils and the amount of water present in samples from farmlands treated with the compound of the invention and conventional untreated farmlands were measured for asparagus, strawberry, and cucumber crops. Measurements were performed both in dry conditions, at time t = 0 h, and in wet soils after 24 h.

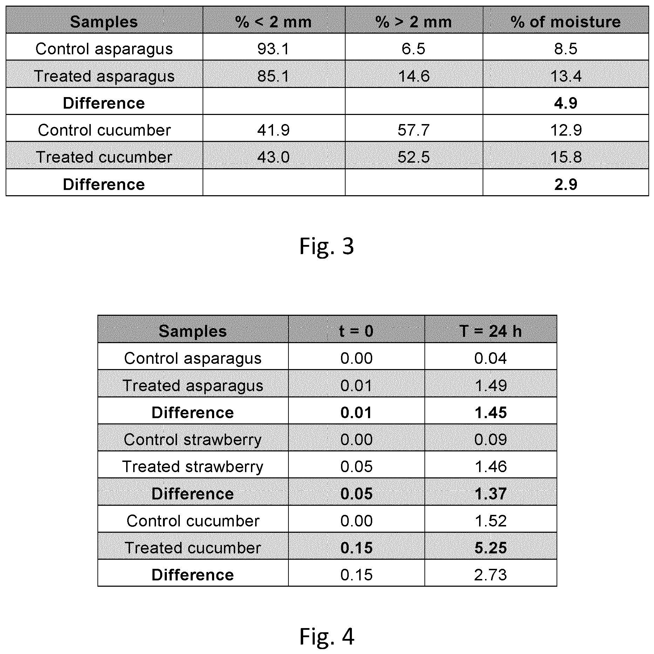

Based on the results shown in Figure 3, the higher wetting capacity of the sample of treated asparagus with respect to untreated control asparagus stands out, with there being a gravimetric difference of 4.9%. This clearly shows that there is an improved structuring of the substrate itself in terms of improvement in water retention capacity. In the case of cucumber, differences in improved cross-linking and structuring are again shown in the case of treated soil with respect to control soil, with a difference in the percentage of moisture of 2.9%.

Figure 4 shows the results of cellular respiration by volume of CO₂, measured in mmol m⁻² s⁻¹. It can be seen at a glance that, in the dry sample (t = 0), clear differences in the concentration of CO₂ can already be observed when comparing the crops, for which the soil has been treated with the compound of the invention, to the control crops, for which the soil has not been treated with said compound. At time zero (t = 0), all the treated samples are superior with respect to their corresponding controls, the case of cucumber being particularly significant with a difference before wetting of 0.15 mmol m⁻² s⁻¹. Those differences are even greater after 24 hours of incubation, with differences of 1.45 and 1.37 mmol m⁻² s⁻¹ for asparagus and strawberry, respectively, and with a much more pronounced difference of 2.73 mmol m⁻² s⁻¹ for the case of cucumber.

The differences observed in gravimetric moisture indicate that treated soils present greater cross-linking, and therefore greater water retention capacity. This different percentage of moisture that treated soil is capable of absorbing with respect to conventional soil is the direct consequence of the type of structure existing in the soil. While the soil in which the compound of the invention has been administered has a segmented block- or sheet-type structure, conventional soil has a granular structure, which causes water to permeate quickly with barely any accumulation capacity.

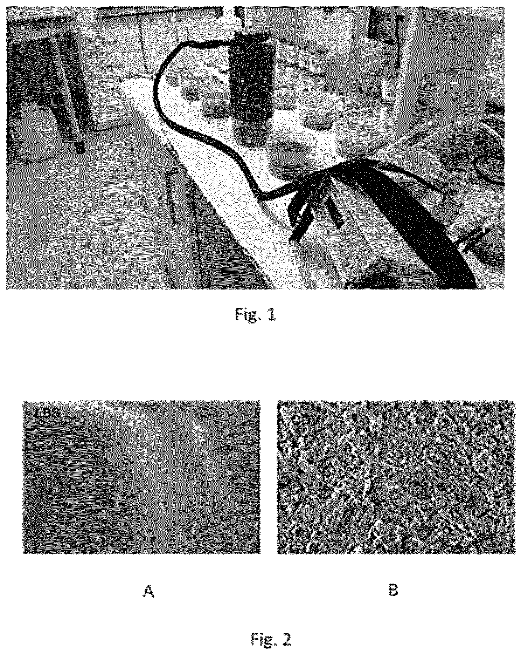

Figure 2 shows two scanning electron microscopy images in which the characteristics of both soils are shown. The image on the left corresponds with granular soil, whereas the image on the right shows a more segmented block-like structure. The magnification of both photographs is the same and the so very different type of structure is clearly observed. This morphological difference suggests that the parameters of cellular respiration are also very different, since the water accumulation capacity is very different, and therefore microbial growth capacity must also be very different, since the latter depends on factors such as the presence of water to a greater or lesser extent, or the presence of bacteria which generate metabolites that promote the development of the bacterial flora. In fact, the parameters of CO₂ cellular respiration acquired experimentally as a measurement of microbial growth both in dry conditions and in wet soil, like throughout the incubation process, show precisely this tendency.

Treated soils present higher respiration at all times, but it is much more pronounced in soil where cucumber had been grown. As for the asparagus and strawberry soil samples, very similar results were obtained, all showing higher carbon concentration in treated soil samples than in conventional samples.

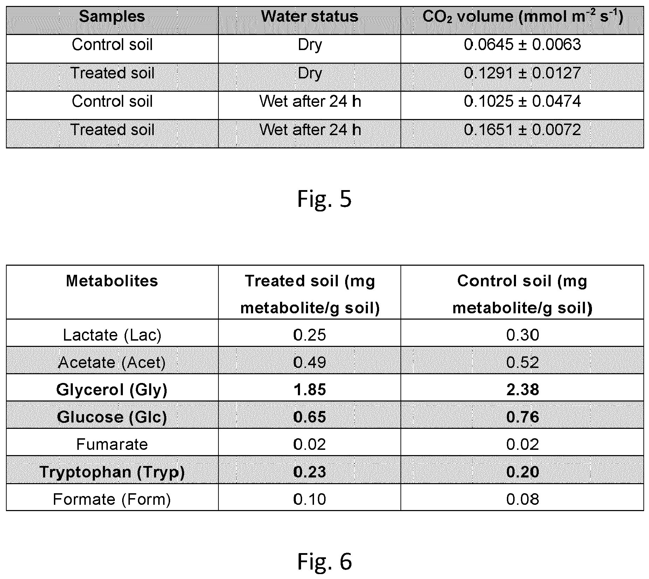

In Example 3, the amount of carbon fixed in farmlands treated with the compound of the invention and conventional untreated farmlands were measured for baby leaf lettuce crops. Measurements were performed both in dry conditions and in wet soils after 24 h. The results can be seen in Figure 5.

When comparing dry samples, the farmland treated with the compound of the present invention reports an increase in CO₂ absorption of more than 100% compared to the control soil. In terms of wet samples, this difference is less pronounced; however, even so, the treated soil absorbs 61% more CO₂ than the control soil. When this data is extrapolated to tons of CO₂ per hectare per year, it can be seen that in soils such as those of Example 3 treated with the compound of the invention 2.30 tons of CO₂ can be fixed, whereas in soils such as those of Example 3 which are not treated, only 1.42 tons are fixed per year per hectare.

In Example 4, the amount of carbon fixed in soil, the increase of water retention in soil, and the root development (by weight) of the vegetables grown in farmlands treated with the compound of the invention and conventional farmlands which are not treated were measured for lettuce, cucumber, strawberry, and asparagus crops.

For cucumber, a 17.5% increase in root development, a 173% increase in carbon fixation, and a 2.9% increase in water retention were observed in treated soils with respect to control soils. For strawberry, a 22% increase in root development and a 242% increase in carbon fixation were observed in soils treated with the compound of the invention. Finally, for asparagus, a 268% increase in carbon fixation and a 4.9% increase in water retention were observed in treated soils with respect to untreated soils.

In this manner, it can be seen that the increase in carbon capture is associated with root growth. Furthermore, in the case of asparagus, a significant regeneration of the beneficial microbiota was also observed, thereby displacing *Fusarium oxysporum,* which is a fungal species that causes vascular wilt.

In Example 5, the amount of metabolites present in soil samples treated with the compound of the invention and untreated or control samples was measured for lettuce crops, Baby leaf variety.

As can be seen in Figure 6, there are marked metabolomic differences between the treated soil and the soil in which only conventional management (control) was followed, specifically 3 metabolites stand out for their relevance, which are glycerol, glucose, and tryptophan. Glycerol is found in higher proportions in conventional soil, and this causes a decrease in native populations of aerobic microbiota, mycorrhizae, and nitrogen-fixing bacteria. Glucose levels are also elevated in this soil, which promotes the possibility of the development of vascular pathogens such as *Fusarium.* In the treated soil, tryptophan values that reach values beyond normal values are observed, this being a precursor of indoleacetic acid that favors the development of crop roots.

For the reasons set forth in the examples, a first aspect of the invention relates to a composition, preferably an aqueous or lyophilized composition, comprising the bacterium *Microbacterium maritypicum.* As explained above in the state of the art, the cellular respiration of certain bacteria present in soil helps to fix carbon in said soil by absorbing CO₂ from the air and emitting oxygen, and the examples show that CO₂ absorption in farmlands increases significantly when these farmlands are treated with a compound comprising the bacterium *Microbacterium maritypicum.* Said bacterium, as can be seen in Example 5, alters soil metabolomics, such that the amount of metabolites associated with vascular pathogens and with the reduction of aerobic microbiota, mycorrhizae, and nitrogen-fixing bacteria, such as glycerol or glucose, is reduced, and metabolites having a probiotic effect, favoring the development of bacterial populations capable of enriching the soil with different metabolites, such as with tryptophan, for example, are increased. This favors the development of the microbiota present in the soil, which ultimately allows fixing more carbon in said soil by means of cellular respiration, such that CO₂ is absorbed and incorporated in the bacteria.

The bacterium *Microbacterium maritypicum* can be obtained from cereal root tissue (see Example 1) by means of the gradient dilution method, separating cells of the cereal root tissue by means of microbiological classification and PCR identification. Alternatively, the bacterium *Microbacterium maritypicum* can also be obtained through depositories accessible to the public. In particular, it can currently be found at least in the depositories of the list comprising: DSMZ under DSM number 12512, ATCC under number 19260, IFO under number 15779, NCIMB under number 1050, NBRC under number 15779, and NRRL under number B-24223. However, it should be noted that the bacterium *Microbacterium maritypicum* may also be obtained from other banks or through other methods not described herein but accessible to one skilled in the art.

Advantageously, the introduction of the bacterium *Microbacterium maritypicum* improves the absorption of carbon in soil, which has a positive impact on the environment, reduces carbon footprint, reduces global warming and, not only that, also leads to a greater root and aerial development of the plant, increasing the amount of production for one and the same farming area. The introduction of the bacterium *Microbacterium Maritypicum* also increases water capture, which advantageously allows taking better advantage of the watering volume and saving water and natural resources, which has a beneficial effect economically and for the environment.

In a preferred embodiment of the first aspect of the invention, the compound of the invention (used in Examples 2, 3, 4, and 5) comprises the bacterium *Microbacterium maritypicum* in an amount of between 10 and 10¹² colony-forming units per gram (cfu/g). Preferably, the compound of the present invention comprises the bacterium *Microbacterium maritypicum* in an amount of between 1.1×10² and 5.6×10⁸ cfu/g. Even more preferably, it comprises the bacterium *Microbacterium maritypicum* in an amount of between 1×10³ and 1×10⁸ cfu/g.

It should be noted that the mentioned amounts of colony-forming units per gram may vary with a margin of 30%, i.e., for each interval of cfu/g, there can be 30% more or 30% less with respect to what is indicated above. Preferably, this margin is ±20%, more preferably ±10%, even more preferably ±5%.

Advantageously, this amount of colony-forming units per gram favors the development of the population of *Microbacterium maritypicum* once this is applied on soil, promoting an increase in the fixation of carbon in soil in an effective and measurable manner.

In a more preferred embodiment of the first aspect of the invention, the composition of the invention comprises an aqueous medium. Preferably, the aqueous medium is sterilized water. In another preferred embodiment, this aqueous medium can be distilled water. Alternatively, the aqueous medium can be mineralized water or regular water. It should be noted that this aqueous medium can also be extracted by means of drying or lyophilization, and then can be added again.

In a preferred embodiment of the invention, the composition comprises the bacterium *Microbacterium maritypicum,* preferably in an amount of between 10 and 10¹² cfu/g, more preferably between 1×10² and 5.6×10⁸ cfu/g, wherein said bacterium is dissolved or dispersed in a medium characterized in that it comprises one or more of the elements from the list consisting of one or more peptones, extract of one or more yeasts, one or more salts comprising an alkaline metal and a halogen element, one or more salts comprising an alkaline earth metal and a halogen element, and one or more saccharides, preferably monosaccharides or disaccharides, more preferably organic sugar or brown sugar, wherein said elements are dissolved in an aqueous medium such as water. In a preferred embodiment of the present invention, the aqueous medium may comprise mineral water, whereas in another preferred embodiment, the water of the aqueous solution may comprise distilled water. It should be noted that in a preferred embodiment of the present invention the water of the aqueous solution has been sterilized, since this removes microorganisms that are detrimental to and compete with the bacterium *Microbacterium maritypicum* and therefore favors its growth. Preferably, the water has been sterilized by means of the heat application method. It should be noted that in another embodiment of the present invention unsterilized water could be used. Alternatively, the composition comprising the bacterium *Microbacterium maritypicum* and a suitable medium can be lyophilized, dried, or deprived of water by means of any other method, for example, to facilitate their transport and storage. This lyophilization or drying would allow preserving the composition of the invention and subsequently, in a preferred embodiment, adding water to hydrate the composition and to enable applying it in a simple manner by means of, for example, watering techniques. Lyophilization or drying would also allow adding the composition of the invention to composts, fertilizers, or any other solid or liquid medium that will be applied on the soil, preferably on the soil to which water will then be applied.

One or more peptones is understood to mean an enzymatic digest comprising one or more amino acids or amino acid fragments, one or more peptides or small peptide fragments, or the mixture of both. In a preferred embodiment, the type of peptone used in the composition of the invention is peptone from porcine and bovine raw material. It should be noted that one skilled in the art may use other types of commonly used peptones with similar properties. Examples of peptones that can be used in the composition are, but not limited to, peptones of animal origin, peptones from soy, peptones from casein, peptones from potato, peptones from corn, peptones from meat, peptones of bovine origin, peptones of porcine origin, peptones of avian origin, peptones from agar, peptone from yeast, peptones from the liver, peptones from gelatin, peptones from strawberry, peptones from lettuce, peptones from asparagus, or peptones from cucumber, among many others.

Extract of one or more yeasts is understood to mean a mixture of nutrients obtained from yeast, a unicellular fungus, or from a group of yeasts. In a preferred embodiment, the type of yeast used to obtain the extract of yeast of the composition of the present invention is *Saccharomyces cerevisiae.* It should be noted that one skilled in the art may use other types of yeasts known to have suitable nutritional values, and may combine them in a necessary or optimal manner. Examples of the types of yeasts with nutritional properties are, but not limited to, the yeast *Saccharomyces cerevisiae,* used in the preparation of bread, beer, and other food products, the yeast *Candida kefyr,* used in kefir, a fermented milk-based food, the yeast *Candida milleri,* found in bread and beer, or the yeasts *Candida utilis* or *Kluyveromyces fragilis,* which are protein, vitamin, and mineral sources, among many other yeasts.

It should be noted that, preferably, no bactericides or fungicides are applied in the composition.

In another preferred embodiment, the composition of the invention comprises the bacterium *Microbacterium maritypicum,* preferably in an amount of between 10 and 10¹² cfu/g, more preferably between 1×10² and 5.6×10⁸ cfu/g, wherein said bacterium is dissolved or dispersed in a medium characterized in that it comprises one or more peptones, extract of one or more yeasts, sodium chloride, magnesium chloride, calcium chloride, and one or more saccharides, preferably monosaccharides or disaccharides, more preferably organic sugar or brown sugar, wherein said one or more elements are dissolved in an aqueous medium such as water, preferably sterilized water. Alternatively, the composition can be lyophilized, dried, or deprived of water by means of any other method.

Advantageously, the inclusion of one or more of these elements in the medium in which the bacterium *Microbacterium maritypicum* is dissolved or dispersed allows the colonies of bacterium *Microbacterium maritypicum* present in the composition of the invention to develop and grow abundantly, which thereby allows increasing the fixation of carbon in soil, changing the structuring of said soil, improving water retention, and reducing the amount of metabolites that are harmful for crops.

More preferably, the composition comprises the bacterium *Microbacterium maritypicum,* preferably in an amount of between 10 and 10¹² cfu/g, more preferably between 1×10² and 5.6×10⁸ cfu/g, wherein said bacterium is dissolved or dispersed in a medium characterized in that it comprises at least one or more of the elements from the list consisting of one or more peptones, extract of one or more yeasts, sodium chloride, magnesium chloride, calcium chloride, and one or more saccharides, preferably monosaccharides or disaccharides, even more preferably unrefined or whole brown sugar, wherein said one or more elements are dissolved in an aqueous medium such as water, preferably sterilized water. Alternatively, the composition can be lyophilized, dried, or deprived of water by means of any other method. It should be noted that in one embodiment the saccharide is unrefined or whole brown sugar, although in another embodiment it can be refined sugar, or another type of monosaccharides or disaccharides such as, for example, but not limited to, glucose, fructose, galactose, sucrose, or lactose, among many others.

For the salts described in the present invention, it is understood that one skilled in the art may find equivalents with similar chemical properties. In this manner, instead of sodium chloride, it would be possible to carry out the invention with a chloride of another alkaline metal such as, for example, potassium chloride. Similarly, it would be possible to carry out the invention with a salt containing sodium and a halogen element other than chlorine such as, for example, fluorine, bromine, or iodine, where sodium fluoride, sodium bromide, or sodium iodide can thus be used. Similarly, one skilled in the art may find equivalents for the remaining salts according to the families in the periodic table to which they belong such as, for example, equivalents for alkaline earth elements, alkaline elements, metallic elements, non-metallic elements, or halogen elements, among others. In summary, one skilled in the art can use chemical analogs that are known to be biocompatible and are widely used or available in the composition of the present invention.

Advantageously, this particular choice of salts is of great importance to create conditions suitable for the development of *Microbacterium maritypicum* bacterial colonies, which translates into an increase in the amount of atmospheric CO₂ fixed in the soil and other associated benefits described above, where the composition of the present invention has been applied.

In another more preferred embodiment, the composition of the invention comprises the bacterium *Microbacterium maritypicum,* preferably in an amount of between 10 and 10¹² cfu/g, more preferably between 1×10² and 5.6×10⁸ cfu/g, wherein said bacterium is dissolved or dispersed in a medium characterized in that it comprises, in percentage by weight with respect to the total weight of the medium (%w/w):
i. Between 0.06 and 6% of one or more peptones,
ii. Between 0.04 and 4% of extract of one or more yeasts,
iii. Between 0.15 and 15% of one or more salts comprising an alkaline metal and a halogen element,
iv. Between 0.06 and 8% of one or more salts comprising an alkaline earth metal and a halogen element,
v. Between 0.05 and 5% of one or more saccharides, preferably monosaccharides or disaccharides, more preferably brown sugar,
vi. an aqueous medium such as water, preferably sterilized water, until completing 100%.

Alternatively, the composition can be lyophilized, dried, or deprived of water by means of any other method.

Advantageously, this particular choice of salt percentage ranges provides the *Microbacterium maritypicum* bacterial colonies with amounts that are even more suitable for development, which translates into an increase in the amount of atmospheric CO₂ fixed in the soil and other associated benefits described above, where the composition of the present invention has been applied.

In an even more preferred embodiment, the composition of the present invention comprises the bacterium *Microbacterium maritypicum,* preferably in an amount of between 10 and 10¹² cfu/g, more preferably between 1×10² and 5.6×10⁸ cfu/g, wherein said bacterium is dissolved or dispersed in a medium characterized in that it comprises, in percentage by weight with respect to the total weight of the medium (%w/w):
i. Between 0.06 and 6% of one or more peptones,
ii. Between 0.04 and 4% of extract of one or more yeasts,
iii. Between 0.15 and 15% of sodium chloride,
iv. Between 0.07 and 7% of magnesium chloride,
v. Between 0.05 and 5% of one or more saccharides, preferably monosaccharides or disaccharides, more preferably brown sugar,
vi. Between 0.01 and 1% of calcium chloride,
vii. An aqueous medium such as water, preferably sterilized water, until completing 100%.

It should be noted that the presence of one or more peptones and extract of one or more yeasts in the composition is of particular importance so that the bacterium *Microbacterium maritypicum* can obtain the nutrients needed to form colonies and to increase its population. Other compounds of great importance are sodium chloride, magnesium chloride, calcium chloride salts, and sugar, preferably brown or whole sugar. Alternatively, the composition can be lyophilized, dried, or deprived of water by means of any other method.

Advantageously, this particular choice of salts and of the corresponding percentage ranges thereof provide the *Microbacterium maritypicum* bacterial colonies with the combination of salts needed in amounts suitable to enable development, which translates into an increase in the amount of atmospheric CO₂ fixed in the soil and other benefits associated with the proliferation of the bacterium *Microbacterium maritypicum* described above, where the composition of the present invention has been applied.

In another preferred embodiment, the composition of the invention comprises the bacterium *Microbacterium maritypicum,* preferably in an amount of between 10 and 10¹² cfu/g, more preferably between 1×10² and 5.6×10⁸ cfu/g, wherein said bacterium is dissolved or dispersed in a medium characterized in that it consists, in percentage by weight with respect to the total weight of the medium (%w/w), of 0.578% of one or more peptones, 0.385% of extract of one or more yeasts, 1.445% of one or more salts comprising an alkaline metal and a halogen element, 0.771% of one or more salts comprising an alkaline earth metal and a halogen element, 0.482% of one or more saccharides, preferably monosaccharides or disaccharides, more preferably brown sugar, and an aqueous medium such as water, preferably sterilized water, until completing 100% (%w/w). Alternatively, the composition can be lyophilized, dried, or deprived of water by means of any other method. It should be noted that the mentioned amounts can vary with a margin of 30%, i.e., for each element, there can be 30% more or 30% less with respect to what is indicated above. Preferably, this margin is ±20%, more preferably ±10%, even more preferably ±5%. For example, ±20% of 0.578% of one or more peptones means between 0.4624 and 0.6936% of one or more peptones.

Advantageously, these specific salt percentage amounts provide the bacteria *Microbacterium maritypicum* with a medium that is even more suitable for them to reproduce and to better perform their function of fixing CO₂, among others.

In an even more preferred embodiment, the composition of the invention comprises the bacterium *Microbacterium maritypicum,* preferably in an amount of between 10 and 10¹² cfu/g, more preferably between 1×10² and 5.6×10⁸ cfu/g, wherein said bacterium is dissolved or dispersed in a medium characterized in that it consists, in percentage by weight with respect to the total weight of the medium (%w/w), and without considering the weight of the bacterium *Microbacterium maritypicum* itself, of 0.578% of one or more peptones, 0.385% of extract of one or more yeasts, 1.445% of sodium chloride, 0.674% of magnesium chloride, 0.096% of calcium chloride, 0.482% of one or more saccharides, preferably monosaccharides or disaccharides, more preferably brown sugar, and an aqueous medium such as water, preferably sterilized water, until completing 100% (%w/w). Alternatively, the composition can be lyophilized, dried, or deprived of water by means of any other method. It should be noted that the mentioned amounts can vary with a margin of 30%, i.e., for each element, there can be 30% more or 30% less with respect to what is indicated above. Preferably, this margin is ±20%, more preferably ±10%, even more preferably ±5%. For example, ±20% of 0.578% of one or more peptones means between 0.4624 and 0.6936% of one or more peptones.

Advantageously, these specific percentage amounts and percentages of specific compounds provide the bacteria *Microbacterium maritypicum* with a medium that is even more suitable for them to reproduce and to better perform their function of fixing CO₂ in soil, changing the structuring of said soil, improving water retention, and reducing the amount of metabolites that are harmful to crops.

In a preferred embodiment of the invention, the composition comprises the bacterium *Microbacterium maritypicum,* preferably in an amount of between 10 and 10¹² cfu/g, more preferably between 1×10² and 5.6×10⁸ cfu/g, wherein said bacterium is dissolved or dispersed in a medium characterized in that said medium further comprises ferric citrate, sodium sulfate, potassium chloride, sodium bicarbonate, potassium bromide, strontium chloride, boric acid, sodium fluoride, ammonium nitrate, and disodium phosphate, wherein said components are comprised in an aqueous medium such as water, preferably sterilized water. Preferably, the composition comprises the bacterium *Microbacterium maritypicum,* preferably in an amount of between 10 and 10¹² cfu/g, more preferably between 1×10² and 5.6×10⁸ cfu/g, wherein said bacterium is dissolved or dispersed in a medium characterized in that it comprises one or more peptones, extract of one or more yeasts, sodium chloride, magnesium chloride, calcium chloride, one or more saccharides, ferric citrate, sodium sulfate, potassium chloride, sodium bicarbonate, potassium bromide, strontium chloride, boric acid, sodium fluoride, ammonium nitrate, and disodium phosphate, wherein said components are dissolved in an aqueous medium such as water, preferably sterilized water. Alternatively, the composition can be lyophilized, dried, or deprived of water by means of any other method.

Advantageously, adding these salts to the composition facilitates the development of bacterial flora and of the bacterium *Microbacterium maritypicum.*

In a preferred embodiment, the composition comprises the bacterium *Microbacterium maritypicum,* preferably in an amount of between 10 and 10¹² cfu/g, more preferably between 1×10² and 5.6×10⁸ cfu/g, wherein said bacterium is dissolved or dispersed in a medium characterized in that it comprises one or more of the elements from the list consisting of one or more peptones, extract of one or more yeasts, one or more salts comprising a transition metal and citric acid, one or more salts comprising an alkaline metal and a halogen element, one or more salts comprising an alkaline earth metal and a halogen element, one or more salts comprising a non-metal and an alkaline element, one or more salts comprising an alkaline metal and bicarbonate, one or more salts comprising an alkaline metal and a non-metal, one or more acids comprising a metalloid, one or more saccharides, preferably one or more monosaccharides or disaccharides, even more preferably brown sugar, and one or more ammonium compounds, and wherein said one or more elements are dissolved in an aqueous medium such as water, preferably sterilized water. Alternatively, the composition can be lyophilized, dried, or deprived of water by means of any other method.

Advantageously, the inclusion of one or more of these elements in the medium in which the bacterium *Microbacterium maritypicum* is dissolved or dispersed allows the *Microbacterium maritypicum* bacterial colonies present in the composition of the invention to develop and grow abundantly, which thereby allows increasing the fixation of carbon in soil, changing the structuring of said soil, improving water retention, and reducing the amount of metabolites that are harmful for crops.

In another preferred embodiment, the composition comprises the bacterium *Microbacterium maritypicum,* preferably in an amount of between 10 and 10¹² cfu/g, more preferably between 1×10² and 5.6×10⁸ cfu/g, wherein said bacterium is dissolved or dispersed in a medium characterized in that it comprises at least one or more of the elements from the list consisting of one or more peptones, extract of one or more yeasts, ferric citrate, sodium chloride, magnesium chloride, sodium sulfate, calcium chloride, potassium chloride, sodium bicarbonate, potassium bromide, strontium chloride, boric acid, sodium fluoride, ammonium nitrate, one or more saccharides, preferably one or more monosaccharides or disaccharides, even more preferably brown sugar, and/or disodium phosphate, and wherein said one or more elements are dissolved in an aqueous medium such as water, preferably sterilized water. Alternatively, the composition can be lyophilized, dried, or deprived of water by means of any other method.

Advantageously, this particular choice of salts interacts synergistically to create conditions that are more suitable for the development of *Microbacterium maritypicum* bacterial colonies, which translates into an increase in the amount of atmospheric CO₂ fixed in the soil and other associated benefits described above, where the composition of the present invention has been applied.

In another more preferred embodiment, the composition of the invention comprises the bacterium *Microbacterium maritypicum,* preferably in an amount of between 10 and 10¹² cfu/g, more preferably between 1×10² and 5.6×10⁸ cfu/g, wherein said bacterium is dissolved or dispersed in a medium characterized in that it comprises, in percentage by weight with respect to the total weight of the medium (%w/w), and without considering the weight of the bacterium *Microbacterium maritypicum* itself:
i. between 0.1 and 1.2% of one or more peptones,
ii. between 0.01 and 1% of extract of one or more yeasts,
iii. between 0.001 and 0.1% of one or more salts comprising a transition metal and citric acid,
iv. between 0.0001 and 9.003% of one or more salts comprising an alkaline metal and a halogen element,
v. between 0.05 and 3% of one or more salts comprising a non-metal and an alkaline element,
vi. between 0.0002 and 5.18% of one or more salts comprising an alkaline earth metal and a halogen element,
vii. between 0.002 and 0.2% of one or more salts comprising an alkaline metal and bicarbonate,
viii. between 0.0002 and 0.03% of one or more acids comprising a metalloid,
ix. between 0.00005 and 0.002% of one or more ammonium compounds,
x. between 0.0001 and 0.01% of one or more salts comprising an alkaline metal and a non-metal,
xi. optionally between 0.05 and 5% of one or more saccharides, and
xii. an aqueous medium such as water, preferably sterilized water, or until completing 100%.

Alternatively, the composition can be lyophilized, dried, or deprived of water by means of any other method.

Advantageously, this particular choice of salt percentage ranges provides the *Microbacterium maritypicum* bacterial colonies with amounts that are even more suitable for development, which translates into an increase in the amount of atmospheric CO₂ fixed in the soil and other associated benefits described above, where the composition of the present invention has been applied.

In an even more preferred embodiment, the composition of the present invention comprises the bacterium *Microbacterium maritypicum,* preferably in an amount of between 10 and 10¹² cfu/g, more preferably between 1×10² and 5.6×10⁸ cfu/g, wherein said bacterium is dissolved or dispersed in a medium characterized in that it comprises, in percentage by weight with respect to the total weight of the medium (%w/w), and without considering the weight of the bacterium *Microbacterium maritypicum* itself:
i. between 0.1 and 1.2% of one or more peptones,
ii. between 0.01 and 1% of extract of one or more yeasts,
iii. between 0.001 and 0.1% of ferric citrate,
iv. between 0.2 and 8% of sodium chloride,
v. between 0.1 and 5% of magnesium chloride,
vi. between 0.05 and 3% of sodium sulfate,
vii. between 0.01 and 0.15% of calcium chloride,
viii. between 0.005 and 0.5% of potassium chloride,
ix. between 0.002 and 0.2% of sodium bicarbonate,
x. between 0.001 and 0.5% of potassium bromide,
xi. between 0.0002 and 0.03% of strontium chloride,
xii. between 0.0002 and 0.03% of boric acid,
xiii. between 0.0001 and 0.003% of sodium fluoride,
xiv. between 0.00005 and 0.002% of ammonium nitrate,
xv. between 0.0001 and 0.01% of disodium phosphate,
xvi. optionally between 0.05 and 5% of one or more saccharides, preferably one or more monosaccharides or disaccharides, even more preferably brown sugar, and
xvii. an aqueous medium such as water, preferably sterilized water, or until completing 100%.

Alternatively, the composition can be lyophilized, dried, or deprived of water by means of any other method.

For the salts described in the present invention, it is understood that one skilled in the art may find equivalents with similar chemical properties. In this manner, instead of sodium chloride, it would be possible to carry out the invention with a chloride of another alkaline metal such as, for example, potassium chloride. Similarly, it would be possible to carry out the invention with a salt containing sodium and a halogen element other than chlorine such as, for example, fluorine, bromine, or iodine, where sodium fluoride, sodium bromide, or sodium iodide can thus be used. Similarly, one skilled in the art may find equivalents for the remaining salts according to the families in the periodic table to which they belong such as, for example, equivalents for alkaline earth elements, alkaline elements, metallic elements, non-metallic elements, or halogen elements, among others. One skilled in the art can furthermore find analogs of sodium bicarbonate in other known similar compounds with basic pH such as, for example, but not limited to, potassium bicarbonate, sodium carbonate, or calcium carbonate. One skilled in the art can also find analogs of boric acid in similar known acids such as, for example, but not limited to, citric acid, phosphoric acid, or sodium tetraborate. One skilled in the art can also find analogs of ammonium nitrate such as, for example, but not limited to, calcium nitrate, ammonium perchlorate, or urea. In summary, one skilled in the art can use chemical analogs that are known to be biocompatible and are widely used or available in the composition of the present invention. Advantageously, this particular choice of salts and of their corresponding percentage ranges provide the *Microbacterium maritypicum* bacterial colonies with optimum salts in amounts suitable for abundant development, which translates into an increase in the amount of atmospheric CO₂ fixed in the soil and other associated benefits described above, where the composition of the present invention has been applied.

In another preferred embodiment, the composition of the invention comprises the bacterium *Microbacterium maritypicum,* preferably in an amount of between 10 and 10¹² cfu/g, more preferably between 1×10² and 5.6×10⁸ cfu/g, wherein said bacterium is dissolved or dispersed in a medium characterized in that it consists, in percentage by weight with respect to the total weight of the medium (%w/w), and without considering the weight of the bacterium *Microbacterium maritypicum* itself, of 0.5758% of one or more peptones, 0.1248% of extract of one or more yeasts, 0.0144% of one or more salts comprising a transition metal and citric acid, 1.9839% of one or more salts comprising an alkaline metal and a halogen element, 1.0105% of one or more salts comprising an alkaline earth metal and a halogen element, 0.3023% of one or more salts comprising a non-metal and an alkaline element, 0.01919% of one or more salts comprising an alkaline metal and bicarbonate, 0.0024% of one or more acids comprising a metalloid, 0.00017% of one or more ammonium compounds, 0.00096% of one or more salts comprising an alkaline metal and a non-metal and an aqueous medium such as water, preferably sterilized water, or until completing 100%. In an even more preferred embodiment, the composition further comprises 0.5% of one or more saccharides. Alternatively, the composition can be lyophilized, dried, or deprived of water by means of any other method.

It should be noted that the mentioned amounts can vary with a margin of 30%, i.e., for each element from the list, there can be 30% more or 30% less with respect to what is indicated above. Preferably, this margin is ±20%, more preferably ±10%, even more preferably ±5%.

Advantageously, these specific salt percentage amounts provide the bacteria *Microbacterium maritypicum* with a medium that is even more suitable for them to reproduce and to better perform their function of fixing CO², among others.

In an even more preferred embodiment, the composition of the invention comprises the bacterium *Microbacterium maritypicum,* preferably in an amount of between 10 and 10¹² cfu/g, more preferably between 1×10² and 5.6×10⁸ cfu/g, wherein said bacterium is dissolved or dispersed in a medium characterized in that it consists of 0.5758% of one or more peptones, 0.1248% of extract of one or more yeasts, 0.0144% of ferric citrate, 1.9289% of sodium chloride, 0.8637% of magnesium chloride, 0.3023% of sodium sulfate, 0.1439% of calcium chloride, 0.04798% of potassium chloride, 0.01919% of sodium bicarbonate, 0.0067% of potassium bromide, 0.0029% of strontium chloride, 0.0024% of boric acid, 0.00029% of sodium fluoride, 0.00017% of ammonium nitrate, 0.00096% of disodium phosphate, and an aqueous medium such as water, preferably sterilized water, or until completing 100%. In an even more preferred embodiment, the composition further comprises one or more saccharides, preferably one or more monosaccharides or disaccharides, even more preferably brown sugar at 0.5%. Alternatively, the composition can be lyophilized, dried, or deprived of water by means of any other method.

It should be noted that the mentioned amounts can vary with a margin of 30%, i.e., for each element, there can be 30% more or 30% less with respect to what is indicated above. Preferably, this margin is ±20%, more preferably ±10%, even more preferably ±5%. For example, ±20% of 0.578% of one or more peptones means between 0.4624 and 0.6936% of one or more peptones.

Advantageously, these specific percentage amounts and percentages of specific compounds provide the bacteria *Microbacterium maritypicum* with an optimum medium for them to reproduce and to better perform their of fixing CO² in soil, changing the structuring of said soil, improving water retention, and reducing the amount of metabolites that are harmful for crops.

In another preferred embodiment, water has been extracted from the composition of the invention, preferably by means of lyophilization or drying. Preferably, water has been extracted from the medium in which the bacterium *Microbacterium maritypicum* is dissolved or dispersed, preferably by means of lyophilization or drying. In another preferred embodiment, water can be extracted therefrom by means of evaporation, centrifugation, or other suitable water extraction means. Preferably, the composition comprising the bacterium *Microbacterium maritypicum* and a suitable medium can be lyophilized, dried, or deprived of water by means of any other method, for example, to facilitate their transport and storage. This lyophilization or drying would allow preserving the composition of the invention and subsequently, in a preferred embodiment, adding water to hydrate the composition and to enable applying it in a simple manner by means of, for example, watering techniques. Lyophilization or drying would also allow adding the composition of the invention to composts, fertilizers, or any other solid or liquid medium that will be applied on the soil, preferably on the soil to which water will then be applied. In a preferred embodiment, the lyophilization process includes the freezing step, preferably at a temperature between -60 and - 5°C, more preferably between -50 and -40°C, the sublimation step, in which water is evaporated by applying a vacuum, then the desorption step, in which water is extracted from the lyophilization chamber used to that end, and subsequently the sealing of the composition under vacuum. It should be noted that one skilled in the art may know the different ways and parameters suitable for lyophilizing the composition of the invention, such as the drying time, the temperature, or the vacuum level applied. Similarly, one skilled in the art may know different ways to extract water or dry the composition of the invention, for example, by means of arranging said composition on a large surface, such that the contact surface of the composition with air is increased, favoring evaporation. Furthermore, the process can also be accelerated by, for example, but not limited to, adding fans or another mechanism to increase airflow, placing the composition in the open air, or placing it under the sun, among other options, in order to accelerate and facilitate drying.

Advantageously, extracting water from the composition allows substantially reducing the weight of the composition, which is ideal to enable transporting and storing the composition more easily, as well as to increase its service life and/or delay its expiration date. Furthermore, this allows recovering the original composition and/or subsequently diluting it by means of adding an aqueous medium such as water, preferably sterilized water.

A second aspect of the invention relates to the use of the composition of the invention for fixing atmospheric carbon, present in the air in the form of CO₂, in soil. It should be noted that the composition of the invention has both probiotic and prebiotic effects; on one hand, it is suitable for the development of the *Microbacterium maritypicum* bacterial populations which find, in said composition, nutrients and salts needed for them to reproduce and survive, and on the other hand, the nutrients and salts of the composition as well as the metabolites derived from the presence of *Microbacterium maritypicum* act, favoring the growth of microbiota beneficial for crops, while said growth, by means of cellular respiration of the bacteria, favors CO₂ capture. On the other hand, the actual nutrients created by said beneficial microbiota and the presence of the rest of said microbiota at the end of their life cycle entail a regeneration of soil nutrients similar to or better than the application of fertilizer on the soil.

Advantageously, this allows reducing global warming effects associated with higher concentration of atmospheric CO₂, and it also allows structuring the soil in a segmented manner, in blocks, instead of granular, and this allows increasing water retention in soil.

Another preferred embodiment of the second aspect of the invention relates to the use of the composition of the invention for increasing water retention in soil.

Advantageously, increasing water retention in soil allows saving this natural resource, which is good for the environment and furthermore entails cost savings as it also allows the flora present to develop much more, creating soils richer in nutrients.

Another preferred embodiment of the second aspect of the invention relates to the use of the composition of the invention for coating seeds, parts of plants such as the root, stem, or leaves, or as fertilizer. Therefore, the application of the compound of the invention on soil is not limited to direct application or application by means of watering. It can be applied indirectly by applying said composition to the seeds that will be planted. It can also be applied on the plant roots, stems, or leaves before or after planting. On the other hand, the composition can be included in solid fertilizers, fertilized soils, or concentrated liquids.

Advantageously, this way of applying the composition of the present invention in a localized manner allows the plants to benefit optimally from the presence of the bacterium *Microbacterium maritypicum,* both for increased root development and for a higher concentration of beneficial metabolites within the absorption volume of the roots, which translates into increased plant development and more generous harvests.

A third aspect of the invention relates to a method for increasing the fixation of carbon in soil which comprises applying the composition of the invention on said soil. Application on soil can be performed, for example, by means of the incorporating the composition of the invention in irrigation water. The final dilution of the composition of the invention will therefore depend on the irrigation method chosen and on the amount of water required by said method, and which may vary depending on climate conditions, the type of soil, or the type of crop, among other factors.

Advantageously, this method allows the soil to obtain the composition of the invention, which contains the bacterium *Microbacterium maritypicum,* and in this manner the soil can benefit from the presence of said bacterium, increasing the concentration of fixed CO₂, therefore structuring the soil, and improving the amount of nutrients in the soil and the quality of the harvests.

Another preferred embodiment of the third aspect of the invention relates to a method for increasing water retention in soil, which comprises applying on said soil the composition of the invention. Application on soil can be performed, for example, by means of incorporating the composition of the invention in irrigation water. The final dilution of the composition of the invention will therefore depend on the irrigation method chose and on the amount of water required by said method, which may vary depending on climate conditions, the type of soil, or the type of crop, among other factors. It should be noted that water retention may refer to retention of irrigation water, retention of rainwater, or retention of moisture derived from closeness to bodies of water such as a river or underground water.

Advantageously, this method allows the soil to obtain a more segmented structure that allows increasing water retention in soil, which is beneficial because it means lower water expenditure with the subsequent cost savings and benefit for the ecosystem.

Another preferred embodiment of the third aspect of the invention relates to a method for increasing the fixation of carbon in soil or the retention of water in soil, wherein the application of the composition on said soil is performed in an amount preferably of between 1 and 100 liters per hectare, more preferably between 2 and 50 liters per hectare, even more preferably between 10 and 30 liters per hectare. It should be noted that if the composition of the invention were lyophilized, application on said soil would preferably be performed in an amount of between 4 and 400 grams per hectare, more preferably between 8 and 200 grams per hectare, even more preferably between 40 and 120 grams.

Advantageously, this amount of composition is optimal for obtaining the number of bacteria and nutrients suitable for developing a flora beneficial for the soil, in order to capture and fix more CO₂ in soil, among other benefits.

A more preferred embodiment of the third aspect of the invention relates to a method for increasing the fixation of carbon in soil or the retention of water in soil, wherein the application of the composition on said soil is performed in an amount of between 1 and 100 liters per hectare, preferably between 2 and 50 liters per hectare, more preferably between 10 and 30 liters per hectare, wherein prior to the application of said composition on soil, this composition is diluted in between 1000 and 100000 liters of water, preferably diluted in between 3000 and 30000 liters of water. This embodiment allows applying the composition of the invention allows using common irrigation methods. It should be noted that these irrigation methods may be highly varied. For example, in one embodiment, between 10 and 30 liters of the composition can be diluted in 3000 liters of water, and they can be applied on the soil with the help of barrels and tractors. These amounts may vary by adding more or less liters of the composition or more or less liters of water. In another example of an embodiment, between 10 and 30 liters of the composition can be diluted in 30000 liters of water, and a sprinkler irrigation system can be used to apply them on soil homogeneously. Again, these amounts may vary by adding more or less liters of the composition of the invention or more or less liters of water. It should be noted that one skilled in the art can use different ways of watering on different surfaces, and therefore the dilution amounts will vary accordingly.

It should be noted that, preferably, neither bactericides nor fungicides are applied in the composition, or in the water used in the dilution thereof, or in the mixture of both.

Advantageously, this dilution allows applying the composition of the invention together with irrigation water, thereby facilitating a homogenous application, as well as favoring a wet soil that allows the bacterium *Microbacterium maritypicum* and the rest of the microbiota to benefit from the presence of this bacterium, to have access to water so as to be able to grow and reproduce. This method for increasing the fixation of carbon in soil is therefore optimum for developing a flora beneficial for the soil, in order to capture and fix more CO₂ in soil, among other benefits.

Another preferred embodiment of the third aspect of the invention relates to a method for increasing the fixation of carbon in soil or the retention of water in soil, wherein application on said soil is performed by an irrigation method consisting of watering, spray irrigation, gravity irrigation, drip irrigation, flood irrigation, irrigation by means of cisterns and tractors, irrigation by means of sprinklers, or underground irrigation, among others. It should be noted that one skilled in the art may know or use other ways of irrigation and can therefore apply the composition of the invention in irrigation water and apply same as is normally done.

Advantageously, this method allows readily applying the composition of the invention, adding it to the suitable irrigation method.

Another preferred embodiment of the third aspect of the invention relates to a method for increasing the fixation of carbon in soil or the retention of water in soil, comprising at least one or more of the steps from the list consisting of coating a plant seed with the bacterium *Microbacterium maritypicum,* coating a part of the plant with said bacterium, spraying said bacterium on a part of the plant, spraying said bacterium on the roots of the plant, spraying said bacterium in a furrow in which a plant or seed will be placed, soaking said bacterium in a part of the plant or in an area in which a plant will be placed, propagating said bacterium in a part of the plant or in an area in which a plant will be placed, transmitting said bacterium in a part of the plant or in an area in which a plant will be placed.

Advantageously, this allows applying the composition of the invention in a localized manner and closer to the plants that will benefit from the effects that said composition has on the soil in its vicinity.

### Examples

### EXAMPLE 1. Preparation of the compound of the invention

In one embodiment, the compound of the present invention comprises a *Microbacterium maritypicum* bacterial culture and an aqueous solution.

The bacterial culture can be obtained both from depositories accessible to the public (DSMZ DSM number 12512, ATCC number 19260, IFO number 15779, NCIMB number 1050, NBRC number 15779, and NRRL number B-24223) and by following the bacterial isolation, filtration, and classification method below:
Several bacterial strains are isolated from cereal root tissue by means of the gradient dilution method. The cereal root tissue is prepared by sieving it with the culture, and microorganism strains are obtained from a selection of endophytes, from which the bacterial strain is filtered by means of microbiological PCR classification and identification, thereby obtaining *Microbacterium maritypicum.*

Said bacterium is present in the compound of the invention in an amount which may vary between 1.1×10² and 5.6×10⁸ colony-forming units per gram (cfu/g).

With respect to the aqueous solution, in one embodiment, the formula below is followed:

| | |
|---|---|
| Peptone | 6.0 g |
| Extract of yeast | 4 g |
| Sodium chloride | 15 g |
| Magnesium chloride | 7 g |
| Calcium chloride | 1 g |
| Whole sugar | 5 g |
| Sterilized water | 1000.0 ml |

Where the aqueous medium is inside an autoclave working at 121°C and rotating continuously, even during dispensing.

Bacterial peptone obtained from porcine and bovine raw material is used as peptone. Said peptone is an enzymatic digest of animal origin used as ingredient in the culture medium.

Extract of *Saccharomyces cerevisiae* yeast is used as extract of yeast. Said yeast is a concentrate of the water-soluble portion of yeast cells of several *Saccharomyces cerevisiae* strains, particularly cultured in a molasses-based medium, which have been subjected to autolysis.

In another preferred embodiment, the formula below is followed to obtain the aqueous solution of the composition of the invention:

| | |
|---|---|
| Peptone | 6.0 g |
| Extract of yeast | 1.3 g |
| Ferric citrate | 0.15 g |
| Sodium chloride | 20.1 g |
| Magnesium chloride | 9.0 g |
| Sodium sulfate | 3.15 g |
| Calcium chloride | 1.5 g |
| Potassium chloride | 0.50 g |
| Sodium bicarbonate | 0.20 g |
| Potassium bromide | 0.07 g |
| Strontium chloride | 0.03 g |
| Boric acid | 0.025 g |
| Sodium fluoride | 0.003 g |
| Ammonium nitrate | 0.0018 g |
| Disodium phosphate | 0.01 g |
| Sterilized water | 1000.0 ml |

Where the aqueous medium is inside an autoclave working at 121°C and rotating continuously, even during dispensing. Where, furthermore, alternatively up to 5 grams of brown sugar can be added.

Bacterial peptone obtained from porcine and bovine raw material is used as peptone. Said peptone is an enzymatic digest of animal origin used as ingredient in the culture medium.

Extract of *Saccharomyces cerevisiae* yeast is used as extract of yeast. Said yeast is a concentrate of the water-soluble portion of yeast cells of several *Saccharomyces cerevisiae* strains, particularly cultured in a molasses-based medium, which have been subjected to autolysis.

### EXAMPLE 2. Results of assays with the compound of the invention.

Measurements of the amount of carbon absorbed in soil and the amount of water present in the soil for samples from farmland treated with the compound of the invention of Example 1 and samples from untreated or control farmland, by means of respirometry with infrared CO₂ sensor, as can be seen in Figure 1, and measurements of gravimetric moisture, respectively, were performed. The amount of carbon dioxide absorbed is proportional to the volume of bacterial respiration and therefore proportional to the biological activity in the soil sample.

The crops in the soil samples analyzed were asparagus, strawberry, and cucumber, both for crops with treated soil and for crops with untreated soil.

To perform this assay, the sample was first sieved to obtain soil with a fraction having a grain diameter below 2 mm. The remaining fractions above or below 2 mm (see Figure 3) were calculated in percentage, and the soils were then left to dry at room temperature. Coupling cylinders of the CO₂ sensor arm as shown in Figure 1 were prepared. Next, samples were taken at time 0 for dry sample, and a gravimetrically controlled wetting was then performed to calculate the moisture content of said soil. The wetting process was performed slowly to prevent oversaturations of the soil. The soil samples were then subjected to an incubation process to estimate microbial growth evolution through the measurement of CO₂, and finally respirometry readings were taken at 0 and 24 hours.

Figure 3 shows the results of the different percentages by weight of the fractions above and below 2 mm. On one hand, it shows the gravimetric moisture expressed in % (g H₂O/100 g soil) needed for a homogenous humectation of the soil where reading and incubation were performed, for soil samples where asparagus and cucumbers were grown.

Figure 4 shows the results of CO₂ cellular respiration (in mmol·m⁻²·s⁻¹) in dry soils, at time 0, and in homogenously wetted soils after leaving them to incubate for 24 hours, for soil samples where asparagus, strawberry, and cucumber were grown.

### EXAMPLE 3. Results of assays with the compound of the invention in baby leaf lettuce

Measurements of the amount of carbon absorbed by samples from farmland treated with the compound of the invention at a dose of 10 l/ha and samples from untreated or control farmland, by means of respirometry with infrared CO₂ sensor, were performed. The amount of carbon dioxide absorbed is proportional to the volume of bacterial respiration and therefore proportional to the biological activity in the soil sample.

The crops in the soil samples analyzed were baby leaf lettuce, taking place in Malaga in April 2022, both for crops with treated soil and for crops with untreated soil or control. Coupling cylinders of the CO₂ sensor arm as shown in Figure 1 were prepared. Next, samples were taken at time 0 for dry sample, and a gravimetrically controlled wetting was then performed to calculate the moisture content of said soil. The wetting process was performed slowly to prevent oversaturations of the soil. The soil samples were then subjected to an incubation process to estimate microbial growth evolution through the measurement of CO₂, and finally respirometry readings were taken at 0 and 24 hours. The results can be seen in Figure 5.

### EXAMPLE 4. Result of the field application of the compound of the invention.

Measurements of the amount of carbon absorbed by samples from farmland treated with the compound of the invention of Example 1 at a single dose of 20 l/ha and samples from conventional untreated or control farmland, by means of respirometry with infrared CO₂ sensor, Were performed. Measurements of the length of the root development were also performed.

The crops in the soil samples analyzed were cucumber, strawberry, and asparagus, both for crops with treated soil and for crops with untreated soil.

Cucumber was grown in Campohermoso, Almeria in 2021. Neither systemic fungicides nor bactericides were used. A 17.5% increase in root development (by weight) was observed. In terms of soils, a 173% increase in carbon fixation and a 2.9% increase in water retention in treated soils with respect to control soils stand out.

Strawberry was grown in Moguer, Huelva, in 2021. A clear difference was observed in terms of aerial and root vigor between the plants in which said protocol was followed (1 single application of the compound of the invention of Example 1 at a dose of 20 l/ha) and in which conventional management was followed. In the treated strawberry plant, a 22% increase in the root system and a 242% increase in carbon capture were generated.

Asparagus was grown in Extremadura in 2021. It was observed that, in the crop treated with the compound of the invention of Example 1 at a dose of 20 l/ha, without using systemic fungicides or bactericides, carbon capture increased by 268% in the soil, and water retention increased by 4.9%, with respect to the soil in which conventional crop management was followed. In this plot, there was a significant regeneration of beneficial microbiota, thereby displacing *Fusarium oxysporum,* which is a fungal species that causes vascular wilt.

### EXAMPLE 5. Soil metabolomics study.

Metabolomics measurements were performed on soil samples from a lettuce crop, Baby leaf variety, grown in Malaga in 2022. A Bruker Avance III 600 spectrophotometer, equipped with a temperature-controlled SampleJet autosampler with up to 480 positions, was used in this study.

Figure 6 reflects different variations in different values that make up the metabolome of the soil where Baby leaf lettuce crops were grown, in which a conventional management was followed and another one which resorted to the use of the compound of the invention of Example 1 at a dose of 10 l/ha.

## Claims

1. A composition suitable for increasing the fixation of carbon in soil comprising the bacterium *Microbacterium maritypicum.*

2. The composition according to claim 1, wherein the bacterium *Microbacterium maritypicum* is present in an amount of between 1×10² and 5.6×10⁸ cfu/g.

3. The composition according to claim 1 or 2, comprising the bacterium *Microbacterium maritypicum,* preferably in an amount of between 1×10² and 5.6×10⁸ cfu/g, wherein said bacterium is dissolved or dispersed in a medium, **characterized in that** it comprises one or more peptones, extract of one or more yeasts, sodium chloride, magnesium chloride, calcium chloride, and one or more saccharides, wherein said components are comprised in an aqueous medium such as water, preferably in sterilized water.

4. The composition according to claim 3, wherein said medium in which the bacterium *Microbacterium maritypicum* is dissolved or dispersed, preferably in an amount of between 1×10² and 5.6×10⁸ cfu/g, comprises, in percentage by weight with respect to the total weight of the medium (%w/w):
i. between 0.06 and 6% of one or more peptones,
ii. between 0.04 and 4% of extract of one or more yeasts,
iii. between 0.15 and 15% of sodium chloride,
iv. between 0.07 and 7% of magnesium chloride,
v. between 0.05 and 5% of one or more saccharides,
vi. between 0.01 and 1% of calcium chloride, and
vii. an aqueous medium such as water, preferably sterilized water, until completing 100%.

5. The composition according to claim 4, wherein said medium in which the bacterium *Microbacterium maritypicum* is dissolved or dispersed, preferably in an amount of between 1×10² and 5.6×10⁸ cfu/g, comprises ±20% of 0.578% of one or more peptones, ±20% of 0.385% of extract of one or more yeasts, ±20% of 1.445% of sodium chloride, ±20% of 0.674% of magnesium chloride, ±20% of 0.096% of calcium chloride, ±20% of 0.482% of one or more saccharides, and an aqueous medium, preferably sterilized water, until completing 100% (%w/w).

6. The composition according to claim 3, wherein said medium in which the bacterium *Microbacterium maritypicum* is dissolved or dispersed, preferably in an amount of between 1×10² and 5.6×10⁸ cfu/g, further comprises ferric citrate, sodium sulfate, potassium chloride, sodium bicarbonate, potassium bromide, strontium chloride, boric acid, sodium fluoride, ammonium nitrate, and disodium phosphate, wherein said components are comprised in an aqueous medium such as water, preferably sterilized water.

7. The composition according to claim 6, wherein said medium in which the bacterium *Microbacterium maritypicum* is dissolved or dispersed, preferably in an amount of between 1×10² and 5.6×10⁸ cfu/g, comprises in percentage by weight with respect to the total weight of the medium (%w/w):
i. between 0.1 and 1.2% of one or more peptones,
ii. between 0.01 and 1% of extract of one or more yeasts,
iii. between 0.001 and 0.1% of ferric citrate,
iv. between 0.2 and 8% of sodium chloride,
v. between 0.1 and 5% of magnesium chloride,
vi. between 0.05 and 3% of sodium sulfate,
vii. between 0.01 and 0.15% of calcium chloride,
viii. between 0.005 and 0.5% of potassium chloride,
ix. between 0.002 and 0.2% of sodium bicarbonate,
x. between 0.001 and 0.5% of potassium bromide,
xi. between 0.0002 and 0.03% of strontium chloride,
xii. between 0.0002 and 0.03% of boric acid,
xiii. between 0.0001 and 0.003% of sodium fluoride,
xiv. between 0.00005 and 0.002% of ammonium nitrate,
xv. between 0.0001 and 0.01% of disodium phosphate,
xvi. optionally between 0.05 and 5% of one or more saccharides,
xvii. an aqueous medium such as water, preferably sterilized water, until completing 100%.

8. The composition according to claim 7, wherein said medium in which the bacterium *Microbacterium maritypicum* is dissolved or dispersed, preferably in an amount of between 1×10² and 5.6×10⁸ cfu/g, consists of ±20% of 0.5758% of one or more peptones, ±20% of 0.1248% of extract of one or more yeasts, ±20% of 0.0144% of ferric citrate, ±20% of 1.9289% of sodium chloride, ±20% of 0.8637% of magnesium chloride, ±20% of 0.3023% of sodium sulfate, ±20% of 0.1439% of calcium chloride, ±20% of 0.04798% of potassium chloride, ±20% of 0.01919% of sodium bicarbonate, ±20% of 0.0067% of potassium bromide, ±20% of 0.0029% of strontium chloride, ±20% of 0.0024% of boric acid, ±20% of 0.00029% of sodium fluoride, ±20% of 0.00017% of ammonium nitrate, ±20% of 0.00096% of disodium phosphate, optionally ±20% of 0.5% of one or more saccharides, and an aqueous medium such as water, preferably sterilized water, until completing 100% (%w/w).

9. The composition according to any of claims 1 to 8, wherein water has been extracted from said medium in which the bacterium *Microbacterium maritypicum* is dissolved or dispersed, preferably by means of lyophilization or drying, preferably in an amount of between 1×10² and 5.6×10⁸ cfu/g.

10. Use of the composition according to any of claims 1 to 9 for fixing atmospheric carbon in soil.

11. Use of the composition according to any of claims 1 to 9 for increasing water retention in soil.

12. A method for increasing the fixation of carbon in soil which comprises applying on said soil the composition according to any of claims 1 to 9.

13. The method according to claim 12, wherein between 1 and 100 liters of the composition according to any of claims 1 to 8 are applied on one hectare of soil.

14. The method according to claim 13, wherein the between 1 and 100 liters of the composition according to any of claims 1 to 8 are diluted in between 1000 and 100000 liters of water prior to the application thereof on one hectare of soil, preferably diluted in between 3000 and 30000 liters of water prior to the application thereof on one hectare of soil.

15. The method according to any of claims 12 to 14, wherein the application of the composition on said soil is performed by an irrigation method consisting of watering, spray irrigation, gravity irrigation, drip irrigation, flood irrigation, tractor irrigation, cistern irrigation, sprinkler irrigation, or underground irrigation.
